# EUROPÄISCHE PATENTSCHRIFT

(11) **EP 0 571 958 B1**
(45) Veröffentlichungstag und Bekanntmachung des Hinweises auf die Patenterteilung: **04.09.2002**
(21) Anmeldenummer: 93108446.1
(22) Anmeldetag: 25.05.1993
(51) Int. Cl.: A61F 13/58

(54) **Wegwerfwindeln**
Disposable diapers
Couches-culottes à jeter

(30) Priorität: 26.05.1992 JP 13363492
(43) Veröffentlichungstag der Anmeldung: 01.12.1993
(73) Patentinhaber: UNI-CHARM CORPORATION, Kawanoe-shi Ehime-ken (JP)
(72) Erfinder: Matsushita, Michiyo, Iyomishima-shi, Ehime-ken (JP); Sayama, Yasushi, Kawanoe-shi, Ehime-ken (JP)
(74) Vertreter: Sperling, Rüdiger, Dipl.-Ing.

(56) Entgegenhaltungen:
- EP-A- 0 099 846
- EP-A- 0 292 970

## Beschreibung

Diese Erfindung betrifft allgemein eine Wegwerfwindel und insbesondere Befestigungsmittel, die zum abnehmbaren Verbinden von einander seitlich gegenüberliegenden Seitenabschnitten eines Vorder- und Hinterteiles einer derartigen Windel in der Ebene des Hüftbereiches verwendet werden.

Aus der EP 0 099 846 A1 ist ein Befestigungsmittel an einer Wegwerfwindel bekannt, welches ebenso aus einem Abziehband, einem darübergelegten Befestigungsband sowie einer durch Umfalten des freien Endes des Befestigungsbandes gebildeten Abziehlasche besteht. Im montierten Zustand überragt die nicht klebende Abziehlasche vollständig das vordere Ende des Abziehbande.

Da die umgefaltete Abziehlasche einen wesentlich größeren Querschnitt als das Abziehband aufweist, wird auch bei dieser Anordnung beim Verpressen der Wegwerfwindel und der Befestigungsmittel ein geringerer Druck auf das vordere Ende des Abziehbandes ausgeübt und es besteht ebenso die Gefahr, daß sich dieses beim Abziehen des Befestigungsbandes bzw. beim Tragen ablöst.

Aus der EP 0 292 970 sind ebenso Befestigungsmittel für Wegwerfwindeln bekannt, wobei hier das Befestigungsmittel aus einem einzigen Band besteht, welches gefaltet ist und auf die Oberfläche der Windel aufgeklebt wird. Die obere Hälfte des Bandes bildet das Befestigungsband, wobei dieses an seinem freien Ende über die untere Hälfte des Bandes mit einem nicht überklebenden Bereich übersteht.

Bei diesem Befestigungsmittel ist der nichtklebende Bereich, d.h. die Abziehlasche, nicht durch Umfalten des freien Endes des Befestigungsbandes gebildet. Die Abziehlasche ist somit eine einfache, sehr dünne Schicht, welche etwas über den unteren Bereich des Bandes übersteht und schwer zu ergreifen ist.

Bei dieser Ausbildung der Befestigungsmittel wird der untere Bereich des Bandes, welcher dem separaten Abziehband der vorliegenden Erfindung entspricht, gleichmäßig mit der Oberfläche der Windel verklebt. Im vorderen Bereich, in welchem die Abziehlasche angeordnet ist, erfolgt jedoch keine stärkere Verklebung, so daß auch hier ein Ablösen des Abziehbandes von der Oberfläche der Windel befürchtet werden muß.

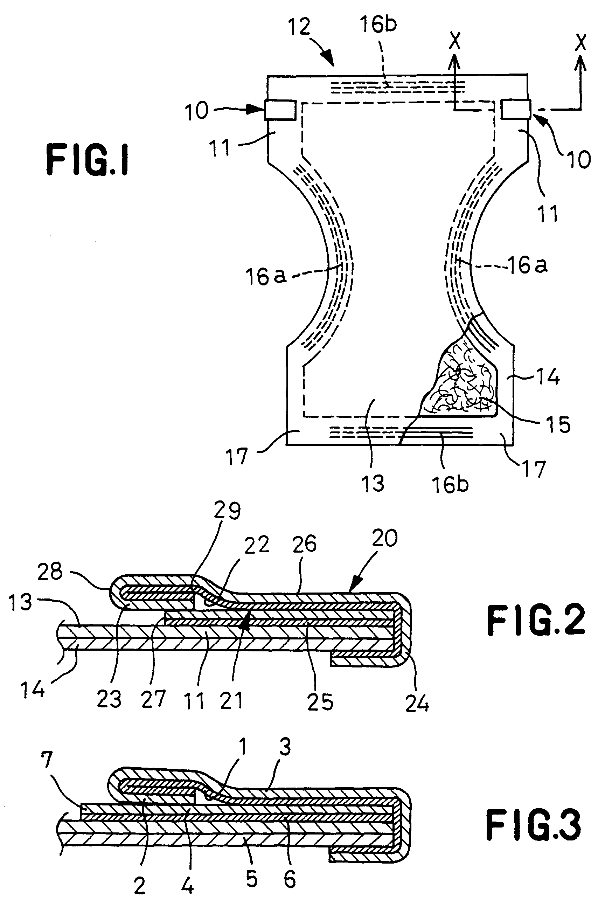

Wie aus Fig. 3 der beiliegenden Zeichnungen ersichtlich ist, sind bekannte Befestigungsmittel so eingerichtet, daß sie jeweils an einander seitlich gegenüberliegenden Seitenabschnitten 5 eines Hinterteiles in der Ebene des Hüftbereiches angebracht sind, wobei sie einerseits ein Befestigungsband 3 mit einer Klebefläche 1 umfassen, die von dessen Innenfläche gebildet ist, und eine an dessen vorderem Ende ausgebildete, nicht klebende Abziehlasche 2, sowie andererseits ein Abziehband 4, auf das die Klebefläche 1 aufgelegt ist und mit dem sie vorübergehend verbunden ist, umfassen. Das Befestigungsband 3 ist so aufgebaut und dimensioniert, daß die nicht klebende Abziehlasche 2 bezüglich dem Vorderende 7 des Abziehbandes 4, das eine klebende Oberfläche 6 aufweist, die an den jeweiligen einander gegenüberliegenden Seitenabschnitten 5 auf der Oberfläche derselben angebracht ist, in einer zurückgezogenen Stellung vorliegt, das heißt, daß das vordere Ende 7 des Abziehbandes 4 über das vordere Ende der nicht klebenden Abziehlasche 2 vorragt, wenn das Befestigungsband 3 auf das Abziehband 4 aufgelegt wurde. Eine derartige Anordnung soll das einfache Abnehmen des Befestigungsbandes 3 vom Abziehband 4 mittels der nicht klebenden Abziehlasche 2, die von den Fingern erfaßt wird, erleichtern.

Wird jedoch ein derartiges Verbundband, das das Befestigungsband 3 umfaßt, dessen Klebefläche 1 auf die Abziehfläche (d.h. die obere Fläche) des Abziehbandes 4 aufgelegt ist, in der Windelfertigungsstraße an der Windel an jedem der einander seitlich gegenüberliegenden Seitenabschnitte 5 des Hüftbereiches angebracht wird, indem das Verbundband, das auf die Windel in diesem Abschnitt aufgelegt wurde, unter Verwendung eines geeigneten Anpreßmittels mit einer glatten Preßoberfläche nach unten gepreßt wird, wird zwischen dem Vorderende 7 und der nicht klebenden Abziehlasche 2 eine Höhendifferenz gebildet, wenn gemäß der verwendeten Konstruktion das Vorderende 7 des Abziehbandes über die nicht klebende Abziehlasche 2 des Befestigungsbandes 3 herausragt. Eine derartige Höhendifferenz führt zu einer unzureichenden Druckausübung auf das Vorderende 7 und in der Folge ist das Vorderende 7 oftmals unzuverlässig an den jeweiligen einander gegenüberliegenden Seitenabschnitten 5 des Hüftbereiches an der Windel befestigt. Eine der Maßnahmen zur Lösung eines derartigen Problemes ist es, ein Anpreßmittel zu verwenden, dessen Anpreßfläche in Übereinstimmung mit der Höhendifferenz abgestuft ausgebildet ist. Auch wenn jedoch ein derartiges Anpreßmittel verwendet wird, kommt es aus verschiedenen Gründen oftmals zu einer Fehlausrichtung des abgestuften Bereiches der Anpreßfläche mit dem abgestuften Bereich des Gegenstückes, beispielsweise, weil das Verbundband eine beträchtliche Flexibilität aufweist und weil die Windelfertigungsstraße die Windelbestandteile einschließlich dieses Verbundbandes mit hoher Geschwindigkeit verarbeiten muß, um eine Massenproduktion mit möglichst geringen Kosten zu erzielen. Demgemäß ist es schwierig, allein durch eine spezielle Ausbildung der Anpreßfläche das Problem zu lösen.

Wie vorstehend beschrieben wurde, wird das vordere Ende 7 des Abziehbandes 4, wenn es unzuverlässig an der Windel angebracht ist, aufgrund der Bewegung der Windel oder dem reibenden Kontakt zwischen dem vorderen Ende 7 und der Haut des Trägers von der Windel abgezogen oder abgehoben, was manchmal zu Problemen wie etwa Ausschlag oder Verkratzen der Haut des Trägers führt. Handelt es sich bei dem Träger um ein neugeborenes Baby, ist dies ein ernsthaftes Problem.

Im Hinblick auf das vorstehend erwähnte Problem ist es die Hauptaufgabe der Erfindung, die Position der nicht klebenden Abziehlasche des Befestigungsbandes und des vorderen Endes des Abziehbandes relativ zueinander zu verändern und dadurch das Problem zu lösen.

### BESCHREIBUNG DER ERFINDUNG

Vorstehend dargelegte Aufgabe wird in Übereinstimmung mit der Erfindung durch eine Wegwerfwindel gelöst, die mit Befestigungsmitteln versehen ist, die zum Verbinden von Vorder- und Hinterteil der Windel an den einander seitlich gegenüberliegenden Seitenabschnitten in der Ebene des Hüftbereichs verwendet werden, wobei jedes der Befestigungsmittel einerseits ein Befestigungsband mit einer Klebefläche umfaßt, die von seiner Innenfläche gebildet ist, und das Befestigungsband an seinem vorderen Ende umgefaltet ist, um eine nicht klebende Abziehlasche zu bilden, sowie andererseits ein Abziehband umfaßt, auf das die Klebefläche aufgelegt ist und vorübergehend mit dieser verklebt ist, dadurch gekennzeichnet, daß die nicht klebende Abziehlasche durch den Aufbau und die Dimensionierung des Befestigungsbandes teilweise über ein vorderes Ende des Abziehbandes vorragt, nachdem das Befestigungsband auf das Abziehband aufgelegt wurde.

Vorzugsweise beträgt die Verbindungsfestigkeit zwischen der Klebefläche des Befestigungsbandes und einer Abziehfläche des Abziehbandes weniger als 150 g.

Das wichtige Merkmal der Erfindung, daß die nicht klebende Abziehlasche des Befestigungsbandes nach vorne über das vordere Ende des Abziehbandes vorragt, mit anderen Worten, daß das vordere Ende unter der Unterfläche der nicht klebenden Abziehlasche in Berührung mit derselben liegt, erlaubt es, das vordere Ende an einer vorgegebenen Stelle mit der Windel mit einer Zuverlässigkeit zu verbinden, die wenigstens gleich derjenigen für den übrigen Teil des Abziehbandes ist, indem das Verbundband, das das Befestigungsband, das auf das Abziehband gelegt ist, umfaßt, von der Oberseite des Befestigungsbandes auf die Windel herabgepreßt wird.

Wenn die nicht klebende Abziehlasche des Befestigungsbandes von der Abziehfläche des Abziehbandes getrennt wird, auf der die Klebefläche vorübergehend aufgeklebt wurde, indem die nicht klebende Abziehlasche von Fingern erfaßt wird, wird das vordere Ende wie auch der übrige Abschnitt des Abziehbandes keinesfalls von der Oberfläche (d.h. Innenfläche) der Windel abgezogen, auf der das Abziehband dauerhaft verklebt bleiben sollte, unabhängig davon, wie schnell oder sogar ruckartig das Befestigungsband vom Abziehband abgezogen wird, nicht nur, da die Klebefläche des Befestigungsbandes nicht mit dem Vorderende des Abziehbandes verklebt ist, sondern auch, weil die Verbindungsfestigkeit (oder die vorübergehende Verklebung) zwischen der Klebefläche und der Abziehfläche so eingestellt ist, daß sie unter einem vorgegebenen Wert liegt.

### KURZE BESCHREIBUNG DER ZEICHNUNGEN

Die Erfindung wird nachfolgend anhand eines Beispiels unter Bezug auf die beiliegenden Zeichnungen im Detail beschrieben, wobei:
- Fig. 1: eine teilweise ausgebrochene Darstellung der Innenseite einer Windel zeigt;
- Fig. 2: eine vergrößerte Schnittdarstellung entlang einer Linie X-X in Fig. 1 zeigt; und
- Fig. 3: eine Schnittdarstellung eines herkömmlichen Befestigungsmittels zeigt.

### BEVORZUGTE AUSFÜHRUNGSFORMEN DER ERFINDUNG

In Fig. 1 ist eine bekannte Wegwerfwindel 12 dargestellt, deren Hinterteil an seinen einander gegenüberliegenden Seitenabschnitten im Hüftbereich jeweils mit Befestigungsmitteln 10 versehen ist. Diese Windel 12 umfaßt eine flüssigkeitsdurchlässige Decklage 13, eine flüssigkeitundurchlässige Außenlage 14, eine zwischen den Lagen 13, 14 gelegte, flüssigkeitsabsorbierende Platte 15, und elastische Elemente 16a, 16b, die um die Beinöffnungen bzw. die Hüftöffnung vorgesehen sind. Dem Fachmann ist bekannt, daß die Befestigungsmittel 10 vorgesehen sind, um die einander seitlich gegenüberliegenden Seitenabschnitte des Hinterteiles, an denen die Mittel 10 angebracht sind, mit den entsprechenden Seitenabschnitten des Vorderteiles zu verbinden.

Wie Fig. 2 zeigt, umfaßt jedes der Befestigungsmittel 10 ein Stück Befestigungsband 20 und ein Stück Abziehband 21 (im folgenden einfach als Befestigungsband bzw. Abziehband bezeichnet). Das Befestigungsband 20, das ein Basisende 24 aufweist, ist an seiner Innenfläche mit einem druckempfindlichen Klebstoff versehen, um eine Klebefläche 22 zu bilden, und an seinem vorderen Ende umgefaltet, um eine nicht klebende Abziehlasche 23 zu bilden. Auf das Abziehband 21 ist ebenfalls auf seiner Innenfläche ein Klebstoff aufgetragen, der die gleiche Wirkung wie der auf das Befestigungsband 20 aufgetragene Klebstoff hat, um so eine Klebefläche 25 zu bilden. Das Abziehband 21 ist fest mit seiner Klebefläche 25 auf die Oberfläche (d.h. Innenfläche) der Decklage 13 an den jeweiligen einander seitlich gegenüberliegenden Seitenabschnitten des Hüftbereiches verbunden. Das Befestigungsband 20 ist fest mit seinem Basisende 24 auf die Rückfläche (d.h. Außenfläche) der jeweiligen einander seitlich gegenüberliegenden Seitenabschnitte 11 des Hüftbereiches aufgeklebt. Die Befestigungsbänder 20 werden jeweils über ihren Hauptteil 26 einschließlich der nicht klebenden Abziehlasche 23 auf das zugehörige Abziehband 21 gefaltet, so daß die Klebefläche 22 auf eine Abziehfläche des Abziehbandes 21, die von dessen Oberfläche gebildet wird, aufgelegt und mit dieser vorübergehend verklebt werden kann. Die Länge des Hauptbereiches 26 ist so bemessen, daß in dieser Stellung die nicht klebende Abziehlasche 23 teilweise über das Vorderende 27 des Abziehbandes 21 vorragt, das heißt, daß das vordere Ende 27 zwischen dem Vorderende und Basisende 28, 29 der nicht klebenden Abziehlasche 23 liegt.

Ist das Vorderende 28 der nicht klebenden Abziehlasche 23 so positioniert, daß es mit dem Vorderende 27 des Abziehbandes 21 fluchtet, ist es schwierig, die nicht klebende Abziehlasche 23 mit den Fingern zu erfassen. Ist das Vorderende 27 des Abziehbandes 21 fluchtend mit oder außerhalb (d.h. rechts) des Basisendes 29 der nicht klebenden Abziehlasche 23 positioniert, wird es nicht nur schwierig sein, die nicht klebende Abziehlasche 23 mit den Fingern zu erfassen, sondern auch die Klebefläche 22 kann mit dem Vorderende 27 des Abziehbandes 21 in Berührung kommen, was es schwierig macht, das Befestigungsband 20 vom Abziehband 21 zu trennen. Weiterhin besteht die Gefahr, daß, in Abhängigkeit von der Verbindungsfestigkeit (oder dem Trennungswiderstand) zwischen der Klebefläche 22 des Befestigungsbandes 20 und der Abziehfläche des Abziehbandes 21 (d.h. den vorübergehend verklebten Oberflächen), das Vorderende 27 des Abziehbandes 21 von der Decklage 13 abgezogen, abgerissen und abgehoben werden kann, wenn auch das Befestigungsband 20 Zug ausgeübt wird, um es vom Abziehband 21 zu trennen.

Die Verbindungsfestigkeit beträgt vorzugsweise weniger als 150 g. Um diese Verbindungsfestigkeit zu messen, wird die nicht klebende Abziehlasche 23 in einer Prüfklemme gehalten und (in der Darstellung in Fig. 2 im Uhrzeigersinn) mit Zugkraft beaufschlagt, um so die Klebefläche 22 des Befestigungsbandes 20, die sich über dessen Hauptteil 26 erstreckt, vom Abzieband 21 abzuziehen. Die Verbindungsfestigkeit entspricht einem Wert der Mindestkraft (g), die erforderlich ist, um die Oberfläche 22 vom Abziehband 21 zu trennen. Soweit dieser Wert weniger als 150 g beträgt, besteht keine Möglichkeit, daß das Vorderende 27 wie auch der übrige Abschnitt des Abziehbandes 21 von der Oberfläche der einander seitlich gegenüberliegenden Seitenabschnitte des Hüftbereiches abgezogen, abgerissen oder abgehoben werden können, auch wenn das Befestigungsband 20 rasch oder sogar ruckartig mit Zug beaufschlagt wird, um so von dem Abziehband 21 getrennt zu werden.

Wie bereits vorstehend erwähnt, werden das Befestigungsband 20 und das Abziehband 21, wie in Fig. 2 dargestellt, in die Windelfertigungsstraße eingeführt, wo das verbundene (laminierte) Band auf die Deck- und Außenlagen 13, 14 aufgelegt wird, die die anderen Komponenten der Windel bilden, und dann an diesen befestigt, indem all diese Komponenten unter Verwendung einer geeigneten Preßeinrichtung miteinander verpreßt werden. In diesem Fall wird auf das Vorderende 27 des Abziehbandes 21 der größte Preßdruck ausgeübt, da die Abschnitte des Bandes 20, 21, die über dem Vorderende 27 des Abziehbandes 21 liegen, eine Gesamtdicke haben, die wesentlich größer ist als die Gesamtdicke der übrigen Abschnitte. Folglich wird das Vorderende 27 fest mit der Decklage 13 der Windel an den einander seitlich gegenüberliegenden Seitenabschnitten 11 des Hüftbereiches verbunden.

Die Werkstoffe für das Befestigungsband 20, das Abziehband 21 und der Klebstoff für die Klebeflächen 22, 25 können aus den gewöhnlich in der Windelindustrie verwendeten Materialien ausgewählt werden. Beispielsweise kann das Befestigungsband 20 aus qualitativ hochwertigem Papier oder einer laminierten Lage, bestehend aus Vliesstoff und Kunststoffolie, bestehen.

Das Abziehband 21 kann aus qualitativ hochwertigem Papier bestehen, dessen obere Fläche (d.h. Abziehfläche) mit Silikonharz beschichtet ist. Der Klebstoff für die Klebeflächen 22, 25 kann sogenannter Heißschmelzkleber sein. Die Werkstoffe für die Decklage 13, die Außenlage 14 und die Platte 15 können ebenfalls aus den herkömmlicherweise in diesem Industriezweig verwendeten Materialien ausgewählt werden. Beispielsweise kann die Decklage 13 aus Vliesstoff oder poröser Kunststoffolie hergestellt sein. Die Außenlage 14 kann aus Kunststoffolie oder einem Laminat, bestehend aus einer derartigen Folie und Vliesstoff hergestellt sein. Die Platte 15 kann aus faseriger oder gebrochener Pulpe, gemischt mit hochabsorbierendem Polymerpulver, hergestellt sein.

Der Aufbau und die Bedienung des Befestigungsbandes, das in der erfindungsgemäßen Windel enthalten ist, erlauben es, die Probleme, wie zum Beispiel Ausschlag, Verkratzen oder ähnliches der Haut des Trägers zu vermeiden, die insbesondere von den Vorderenden des Abziehbandes verursacht werden, wenn dieses abgezogen, abgerissen und von der Decklage abgehoben wird. Demgemäß kann die Windel gemäß der Erfindung auch für neugeborene Babies ohne jegliche Befürchtungen verwendet werden.

Die nicht klebende Abziehlasche ragt über das Vorderende des Abziehbandes vor und bildet einen Spalt zwischen der Lasche und der Decklage der Windel, wodurch die Lasche sehr leicht von den Fingern zu erfassen ist.

## Patentansprüche

1. Wegwerfwindel (12), versehen mit Befestigungsmitteln (10), die zum Verbinden von Vorder- und Hinterteil der Windel an den einander seitlich gegenüberliegenden Seitenabschnitten in der Ebene des Hüftbereiches verwendet werden, wobei jedes der Befestigungsmittel (10) einerseits ein Befestigungsband (20) mit einer Klebefläche (22) umfaßt, die von seiner Innenfläche gebildet ist, und das Befestigungsband (20) an seinem vorderen Ende umgefaltet ist, um eine nicht klebende Abziehlasche (23) zu bilden, sowie andererseits ein Abziehband (21) umfaßt, auf das die Klebefläche (22) aufgelegt ist und vorübergehend mit dieser verklebt ist und **dadurch gekennzeichnet, daß** die nicht klebende Abziehlasche (23) durch den Aufbau und die Dimensionierung des Befestigungsbandes (20) teilweise über ein vorderes Ende des Abziehbandes (21) vorragt, nachdem das Befestigungsband (20) auf das Abziehband (21) aufgelegt wurde.

2. Wegwerfwindel nach Anspruch 1, wobei die Verbindungsfestigkeit zwischen der Klebefläche (22) des Befestigungsbandes (20) und der Abziehfläche des Abziehbandes (21) weniger als 150 g beträgt.

## Claims

1. A disposable diaper (12) provided with fastening means (10) used to bond together front and rear bodies of the diaper at laterally opposite side portions at the level of waist zone, each of said fastening means (10) comprising a fastening tape (20) having an adhesive surface (22) defined by its inner surface, on one hand, the fastening tape being folded at ist front end to form a nonadhesive pick-up lobe (23), and a release tape (21) upon which said adhesive surface (22) is laid and temporarily bonded thereto, on the other hand, and **characterized by** the construction and dimensioning of said fastening tape (20) such that said nonadhesive pickup lobe (23) partially projects forward beyond a front end of said release tape (21) with said fastening tape (20) having been laid upon said release tape (21).

2. A disposable diaper according to claim 1, wherein the bonding strength between the adhesive surface (22) of said fastening tape (20) and the release surface of said release tape (21) is less than 150g.

## Revendications

1. Lange jetable (12) pourvu de moyens de fixation (10) utilisés pour assembler des portions latérales mutuellement opposées d'une partie antérieure et d'une partie postérieure dudit lange, dans le plan de la zone de hanches, chaque moyen de fixation (10) comprenant : d'une part, une bande de fixation (20) dotée d'une face adhésive (22) constituée par sa surface intérieure, et la bande de fixation (20) étant repliée au niveau de son extrémité avant, pour former une languette de détachement (23) non adhésive; et, d'autre part, une bande de détachement (21) sur laquelle est appliquée la face adhésive (22) et qui est provisoirement collée avec cette dernière ; **caractérisé par le fait qu'**en raison de la conception et du dimensionnement de la bande de fixation (20), la languette de détachement (23) non adhésive dépasse partiellement d'une extrémité avant de la bande de détachement (21) après que la bande de fixation (20) a été appliquée sur la bande de détachement (21).

2. Lange jetable suivant la revendication 1, dans lequel l'adhésivité entre la face adhésive (22) de la bande de fixation (20) et la face de détachement de la bande de détachement (21) est inférieure à 150 g.
